(19) Europäisches Patentamt
European Patent Office
Office européen des brevets

(11) **EP 2 311 533 A1**

(12) **EUROPEAN PATENT APPLICATION**

(43) Date of publication:
20.04.2011 Bulletin 2011/16

(51) Int Cl.:
*A63B 23/18* (2006.01)   *A41D 13/00* (2006.01)
*A61B 5/00* (2006.01)    *G06F 3/01* (2006.01)

(21) Application number: 09172859.2

(22) Date of filing: 13.10.2009

(84) Designated Contracting States:
AT BE BG CH CY CZ DE DK EE ES FI FR GB GR HR HU IE IS IT LI LT LU LV MC MK MT NL NO PL PT RO SE SI SK SM TR
Designated Extension States:
AL BA RS

(71) Applicant: **Koninklijke Philips Electronics N.V.**
**5621 BA Eindhoven (NL)**

(72) Inventor: **The designation of the inventor has not yet been filed**

(74) Representative: **Golla-Franz, Anke Lucia**
**Philips**
**Intellectual Property & Standards GmbH**
**Postfach 50 04 42**
**52088 Aachen (DE)**

(54) **Respiration controlling apparatus**

(57)   The invention relates to a respiration controlling apparatus (1) for controlling the respiration of a person. The respiration controlling apparatus comprises a heart rate sensing unit (2) for generating a heart rate signal indicative of the heart rate of the person, a breathing sequence determining unit (3) for determining a desired breathing sequence based on the generated heart rate signal, and a haptic output unit (6, 7, 8) for outputting haptic output signals depending on the determined desired breathing sequence. The respiration controlling apparatus is preferentially used for providing a biofeedforward to a person. Since haptic signals are output, signals like biofeedforward signals can be provided to a person without disturbing another person. This increases the application area of the respiration controlling apparatus.

## FIG. 1

EP 2 311 533 A1

**Description**

**FIELD OF THE INVENTION**

The invention relates to a respiration controlling apparatus, a respiration controlling method and a respiration controlling computer program for controlling the respiration of a person.

**BACKGROUND OF THE INVENTION**

Breathing trainers like the breathing trainer from Sierra Biotechnology comprise a display for instructing a person visually when the person should inhale and when the person should exhale. The breathing trainers are used in applications like yoga, meditation, anti-stress treatments, and blood pressure control. A person can input working parameters like the tidal breath volume, the respiration rate, the inspiration time, the expiration time, the inspiration hold, and the expiration hold. Based on the working parameters a breathing pattern is determined, and the person is visually instructed to inhale and exhale in accordance with the determined breathing pattern.

**SUMMARY OF THE INVENTION**

In a first aspect of the present invention a respiration controlling apparatus for controlling the respiration of a person is presented, wherein the respiration controlling apparatus comprises:

- a heart rate sensing unit for generating a heart rate signal indicative of the heart rate of the person,
- a breathing sequence determining unit for determining a desired breathing sequence based on the generated heart rate signal,
- a haptic output unit for outputting haptic output signals depending on the determined desired breathing sequence.

The above mentioned device of the prior art provides a visual output. However, the use of a visual output is often not suitable for instructing a person, in particular, for providing a biofeedforward. This severely limits the application area. For example, if the device provides a visual signal, for example, a visual biofeedforward, it cannot be used with the eyes of a person closed. Moreover, another person like a bed partner may be disturbed by the visual signal, and the visual signal may keep the person alert. Visual outputs are therefore useable in a limited way only, if the outputs should be used for deep relaxation and sleeping purposes.

The respiration controlling apparatus in accordance with the invention comprises a heart rate sensing unit for generating a heart rate signal, a breathing sequence determining unit for determining a desired breathing sequence based on the generated heart rate signal, and a haptic output unit for outputting haptic output signals depending on the determined desired breathing sequence. Since the haptic output unit is adapted to output haptic output signals, they can be output to a person without disturbing another person. Moreover, the person can close the eyes. This increases the application area of the respiration controlling apparatus.

The respiration controlling apparatus is preferentially used for providing a biofeedforward to a person.

It is preferred that the haptic output unit is adapted to haptically instruct the person to breathe in and to breathe out in accordance with the desired breathing sequence.

It is further preferred that the breathing sequence determining unit is adapted to determine inhale times and exhale times as the breathing sequence, wherein the haptic output unit is adapted to haptically output the inhale times and the exhales times to the person.

For example, the breathing sequence determining unit can be adapted such that the inhale times and the exhale times are defined by a number of heart beats determined from the generated heart rate signal. The breathing sequence can be adapted such that the person should inhale during a first time period defined by a first number of heart beats and that the person should exhale during a second time period defined by a second number of heart beats. The first and second number of heart beats can be predefined, for example, by a manufacturer or by a user. The breathing sequence determining unit can be adapted such that initially predefined first and second numbers of heart beats are used for defining the breathing sequence, wherein the user can modify the first and second numbers of heart beats during operation of the respiration controlling apparatus for adapting the breathing sequence to personal preferences. The first number is, for example, four heart beats defining a time period during which the person should inhale and the second number of heart beats is, for example, five or six heart beats for determining a time period during which the person should exhale. The haptic output unit can be adapted to provide haptic output signals at times defined by a transition between an inhale time period and an exhale time period or the haptic output unit can be adapted to output haptic signals also during the inhale time period and the exhale time period.

It is further preferred that the haptic output unit is adapted to output first haptic signals at the inhale times and second haptic signals at the exhale times, wherein the first and second haptic signals are different.

It is further preferred that the respiration controlling apparatus comprises a coherence determining unit for determining a degree of coherence depending on the generated heart rate signal, wherein the haptic output unit is adapted to provide a haptic output signal depending on the determined degree of coherence.

The haptic output signal, which depends on the determined degree of coherence, can be regarded as a further haptic output signal. Thus, in an embodiment the haptic output signals, which depend on the determined desired breathing sequence, can be regarded as a biofeedforward, in particular, for instructing a person when the person should inhale and exhale, wherein the further output signal provides a biofeedback depending on the determined degree of coherence.

The coherence determining unit is preferentially adapted to determine a heart rate variability (HRV) signal depending on the generated heart rate signal.

The coherence determining unit is preferentially adapted to determine a HRV signal as the time between subsequent heart beats versus time. The coherence determining unit is preferentially further adapted to determine the degree of coherence depending on the determined HRV signal. Preferentially, the degree of coherence is determined depending on the amplitude of the HRV signal. In particular, the degree of coherence increases with increasing amplitude of the HRV signal. A large degree of coherence preferentially indicates a good homeostasis or balance between the parasympathetic and sympathetic nervous system.

The respiration controlling apparatus can be adapted to be switched between a first a mode, in which the haptic output signals depend on the determined desired breathing sequence, and a second mode, in which the haptic output signals depend on the determined degree of coherence. The respiration controlling apparatus can be adapted to allow a user to switch between the first mode and the second mode.

It is further preferred that the respiration controlling apparatus is a handheld apparatus.

It is further preferred that the haptic output unit is adapted to expand and to contract for outputting haptic output signals.

In an embodiment, the haptic output unit is adapted to expand, if the person should inhale, and to contract, if the person should exhale, or vice versa. Moreover, the haptic output unit can be adapted to contract, in particular, to decrease its volume, if the degree of coherence increases, and to extract, in particular, to increase its volume, if the degree of coherence decreases.

It is further preferred that the haptic output unit is adapted to move for outputting a haptic output signal. Preferentially, the haptic output unit is adapted to move in different directions for outputting different haptic signals.

In an embodiment, the haptic output unit is adapted to move in a first direction, if the person should inhale, and to move in a second direction being different to the first direction, if the person should exhale. In use, the first direction can point towards the person and the second direction can point away from the person or vice versa. Moreover, the haptic output unit can be adapted to move in the first direction, if the degree of coherence increases, and to move in the second direction, if the degree of coherence decreases.

It is further preferred that the haptic output unit comprises a movable weight and a weight moving unit for moving the weight such that a balance point of the haptic output unit is modified for moving the haptic output unit. The haptic output unit is preferentially adapted to move the balance point of the haptic output unit in a direction, in which the haptic output unit should move, in particular, in use towards the person or away from the person. This allows moving the haptic output unit in a simple way by just modifying the position of the weight.

It is further preferred that the haptic output unit comprises an outer casing including at least the breathing sequence determining unit. It is further preferred that the outer casing is spherical.

The respiration controlling apparatus is therefore preferentially shaped as a ball, in particular, a ball which can be held in a hand of the person. The ball preferentially includes the moveable weight and the weight moving unit such that the ball can be moved by moving the weight within and relative to the casing for shifting the balance point. This allows the ball to move in a direction, in particular, in a first direction, if the person should inhale or if the degree of coherence increases, and in a second direction, if the person should exhale or if the degree of coherence decreases.

In an embodiment, the haptic output unit is adapted to output temperature signals as haptic output signals. Thus, the haptic output unit preferentially comprises a heating unit and/or a cooling unit for modifying the temperature of the haptic output unit. The change in temperature provides a temperature signal, which allows giving commands to the person haptically, in particular, commands for inhaling and exhaling, or to provide information concerning the degree of coherence. For outputting temperature signals the haptic output unit preferentially comprises a thermo-electric element which transforms electrical energy in thermal energy for heating or cooling a person who touches the haptic output unit. The thermo-electric element can just be a metal element which is heated by resistive heating or another thermo-electric element which can be used for cooling and heating purposes, wherein the haptic output unit can be adapted to switch between cooling and heating by modifying the direction of the current flowing through the thermo-electric element.

In a further aspect of the invention a respiration controlling method for controlling the respiration of a person is presented, wherein the respiration controlling method comprises following steps:

- generating a heart rate signal indicative of the heart rate of the person,
- determining a desired breathing sequence based on the generated heart rate signal,
- outputting haptic output signals depending on the determined desired breathing sequence.

In a further aspect of the invention a respiration controlling computer program for controlling the respiration of a person is presented, wherein the respiration controlling computer program comprises program code means for causing a respiration controlling apparatus as defined in claim 1 to carry out the steps of the respiration controlling method as defined in claim 12, when the computer program is run on a computer controlling the respiration controlling apparatus.

It shall be understood that the respiration controlling apparatus of claim 1, the respiration controlling method of claim 12, and the respiration controlling computer program of claim 13 have similar and/or identical preferred embodiments as defined in the dependent claims.

It shall be understood that a preferred embodiment of the invention can also be any combination of the dependent claims with the respective independent claim.

These and other aspects of the invention will be apparent from and elucidated with reference to the embodiments described hereinafter.

## BRIEF DESCRIPTION OF THE DRAWINGS

In the following drawings:

Fig. 1 shows schematically and exemplarily a cross section of a respiration controlling apparatus,
Fig. 2 shows exemplarily heart beats of a heart rate signal and a corresponding determined desired breathing sequence,
Fig. 3 shows schematically and exemplarily the respiration controlling apparatus held in a hand of a person,
Fig. 4 shows schematically and exemplarily a respiration controlling apparatus in an expanded condition,
Fig. 5 shows schematically and exemplarily the respiration controlling apparatus in a contracted condition,
Fig. 6 shows schematically and exemplarily several units within a compartment of the respiration controlling apparatus,
Fig. 7 shows schematically and exemplarily the respiration controlling apparatus in a contracted condition in a hand of a person,
Fig. 8 shows schematically and exemplarily the respiration controlling apparatus in an expanded condition in the hand of the person, and
Fig. 9 shows a flowchart exemplarily illustrating a respiration controlling method for controlling the respiration of a person.

## DETAILED DESCRIPTION OF EMBODIMENTS

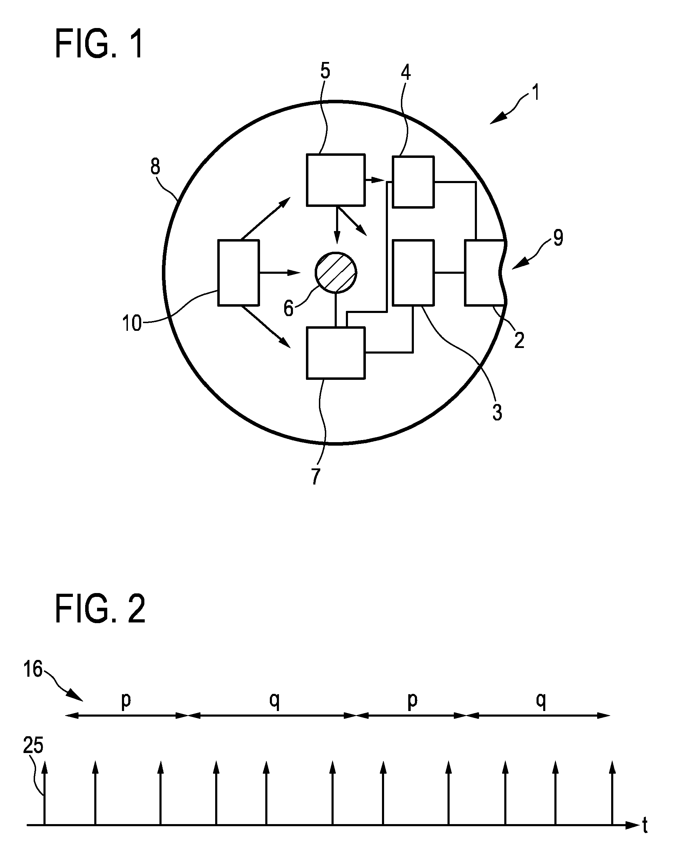

Fig. 1 shows schematically and exemplarily a respiration controlling apparatus 1 for controlling the respiration of a person. The respiration controlling apparatus 1 comprises a heart rate sensing unit 2 for generating a heart rate signal indicative of the heart rate of the person. In this embodiment, the heart rate sensing unit 2 is a PPG sensor with a sensing area 9. The sensing area 9 is adapted to receive a finger of the person for measuring a PPG signal as the heart rate signal.

The respiration controlling apparatus 1 further comprises a breathing sequence determining unit 3 for determining a desired breathing sequence based on the generated heart rate signal. The breathing sequence determining unit 3 is adapted to determine inhale times and exhale times as the breathing sequence. The determination of the inhale times and exhale times will in the following be described with reference to Fig. 2.

Fig. 2 shows the temporal positions of heart beats determined from the generated heart rate signal, and inhale intervals p and exhale intervals q.

The arrows 25 in Fig. 2 indicate temporal positions of heart beats determined from the generated heart rate signal. The inhale intervals p and the exhale intervals q are preferentially defined by a number of heart beats 25. For example, a first number of heart beats can be defined for defining the inhale intervals p and a second number of heart beats can be defined for defining the exhale intervals q. The first number of heart beats and the second number of heart beats can be predefined, for example, by a manufacturer or by a user. In an embodiment, initially a breathing sequence 16 is determined by using a first predefined number of heart beats and a second predefined number of heart beats, wherein during operation of the respiration controlling apparatus the user can modify the first number of heart beats and the second number of heart beats, in order to adapt the breathing sequence to personal preferences. The respiration controlling apparatus can comprise a corresponding user interface for allowing a user to modify the first and second numbers of heart beats.

The first number of heart beats $np$ and the second number of heart beats $nq$ can be integer values or real values, wherein during an inhale time period int(np) heart beats are present and wherein during an exhale time interval int*(nq)* heart beats are present, and wherein int() denotes the integer operation. Since the first and second numbers of heart beats are not restricted to integers, the degree of freedom is increased for the user. As already mentioned above, the first and second numbers of heart beats *np* and *nq* may be chosen individually or even adapted to the person, because generally the appropriate breathing sequence depends on the person's tidal volume and cardiac output.

The transience between the inhale time intervals p and the exhale time intervals q and vice versa are not necessarily coincident with a heart beat. This further increases the degree of freedom. Furthermore, it improves the respiration controlling apparatus also from a physiological point of view. In particular, if the heart beats 25 are derived from a finger PPG signal, there is generally a significant delay between the relevant heart beats in an electrocardiogram signal and in the PPG signal. Preferentially, the inhale time intervals p and the exhale time intervals q vary proportional to the corresponding heart beat activity time axis, hence remaining phase locked. In a simple embodiment, the first number of heart beats *np* and the second number of heart beats *nq* are integers and coincide with the measured heart beats 25.

It should be noted that the heart beats 25 are shown schematically only in Fig. 2, i.e. there can be more or less heart beats present within an inhale time interval p or an exhale time interval q.

The breathing sequence determining unit 3 can be adapted to predict the temporal positions of the heart beats with an auto regressive (AR) filter so that in cases that the measured heart rate signal is unreliable, the AR filter predicts the correct temporal positions of the heart beats, even if the temporal distance between adjacent heart beats varies.

In an embodiment, an adaptive schema for determining initial values for *np* and *nq* can be provided by letting the person breathe in and out in its own pace and determining the breathing pace via the generated heart rate signal. This allows determining initial values for *np* and *nq* .Typical values are, for example, *np* = 2 and *nq* = 3 .As a stating value also values can be used for *np* and *nq* which the person is currently using, wherein these values can be increased gradually.

The breathing sequence determining unit 3 can be adapted such that the heart rate signal does not have to be exactly in sync with respiration. It is sufficient if they are almost in sync. Thus, the desired breathing sequence is preferentially determined such that following equation is fulfilled:

$$\left| n\, \Phi_H - m\, \Phi_R \right| < \varepsilon \,, \qquad\qquad (1)$$

*wherein n and m* are integers, $\Phi_H$ and ($\Phi_R$, are the phases of the heart and respiratory signals respectively, and $\varepsilon$ is a sufficiently small constant number, which is chosen such that the heart rate signal and the desired breathing sequence can be considered as being phase locked.

The respiration controlling apparatus 1 comprises a moveable weight 6 and a weight moving unit 7 for moving the weight 6 such that a balance point of the respiration controlling apparatus is modified for moving the respiration controlling apparatus.

The respiration controlling apparatus further comprises an outer casing 8 being a sphere, wherein the different units of the respiration controlling apparatus 1 are included within this spherical outer casing 8. The respiration controlling apparatus 1 has therefore the shape of a ball. By moving the weight 6 with respect to the casing 8, the ball moves in a corresponding direction. The moveable weight 6, the weight moving unit 7 and the outer casing 8 can be regarded as a haptic output unit.

The haptic output unit 6, 7, 8 is adapted to haptically instruct the person to breathe in and to breathe out in accordance with the determined desired breathing sequence. In particular, the haptic output unit 6, 7, 8 is adapted to haptically output the inhale times and the exhale times to the person. In this embodiment, at the inhale times, the moveable weight 6 is moved such that the respiration controlling apparatus, i.e. in this embodiment, the ball, moves in a first direction, and moves in a second direction being different to the first direction at the exhale times. In use, preferentially the ball moves towards the person at the inhale times and away from the person at the exhale times.

In a preferred embodiment, the weight moving unit is a motor with an excentric weight being the moveable weight, like the vibrator in cell phones.

The haptic output unit can be adapted to output a corresponding haptic output signal during a determined p and q interval or at a transition between p and q intervals.

The respiration controlling apparatus 1 further comprises a coherence determining unit 4 for determining a degree of coherence, wherein the haptic output unit 6, 7, 8 is adapted to provide a haptic output depending on the determined degree of coherence.

In this embodiment, the coherence determining unit 4 determines a HRV signal depending on the generated heart rate signal. The coherence determining unit 4 is adapted to determine the degree of coherence depending on the amplitude of the HRV signal. If the amplitude of the HRV signal is larger, also the degree of coherence is larger, and if the amplitude of the HRV signal is smaller, also the degree of coherence is smaller.

In this embodiment, the respiration controlling apparatus 1 is adapted such that the ball moves in a first direction, if the degree of coherence becomes larger, and in a second direction, if the degree of coherence becomes smaller. In particular, the respiration controlling apparatus 1 is adapted such that in use the ball moves towards the person, if the degree of coherence increases, and away from the person, if the degree of coherence decreases. Thus, the first direction and the second direction point preferentially in opposite directions.

The respiration controlling apparatus 1 further comprises an energy source 5 like a rechargeable battery for providing energy, in particular, electrical energy, to the different units within the outer casing 8, and a control unit 10 for controlling the different units within the outer casing 8.

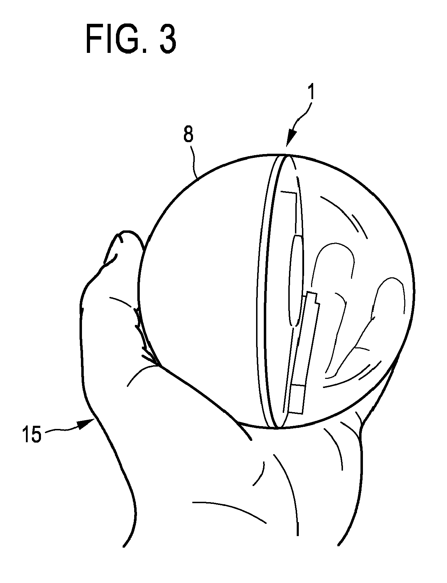

Fig. 3 shows schematically and exemplary the use of the respiration controlling apparatus 1. In use, the respiration controlling apparatus 1 is held by a hand 15 of the person. One of the fingers of the hand 15 is arranged at the sensing area of the respiration controlling apparatus 1 for generating the heart rate signal. The breathing sequence determining unit 3 determines a desired breathing sequence and/or the coherence determining unit 4 determines a degree of coherence, and the respiration controlling apparatus, i.e. in this embodiment the ball, is moved towards or away from the person depending on the determined desired breathing sequence and/or degree of coherence.

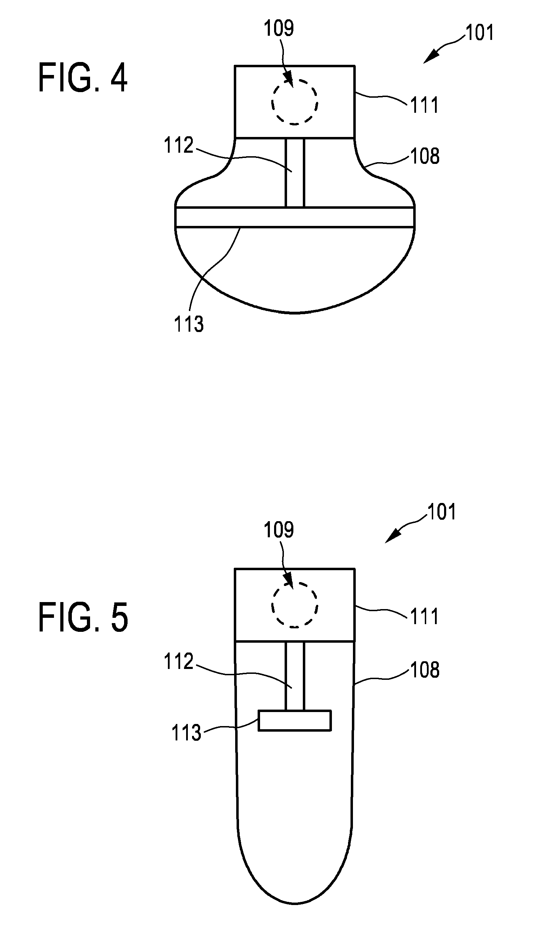

Fig. 4 shows schematically and exemplarily a further embodiment of a respiration controlling apparatus. The respiration controlling apparatus 101 comprises a heart rate sensing unit with a sensing area 109. A finger of a person can be placed at the sensing area 109 for measuring a heart rate signal, in this embodiment, a PPG signal. A breathing sequence determining unit determines a desired breathing sequence based on the generated heart rate signal.

The respiration controlling apparatus 101 comprises a shaft 112 being rotatable and a longitudinal expansion element 113 located at an end of the rotatable shaft 112. The rotatable shaft 112 is connected with a compartment 111 comprising several further units of the respiration controlling apparatus 101, which will be described further below. The longitudinal expansion element 113 and the shaft 112 are surrounded by an elastic outer cover, which is stretched and expanded, if the longitudinal expansion element 113 is in a first rotational position as shown in Fig. 4, and which is not stretched and expanded, i.e. which has a smaller contracted shape, if the longitudinal expansion element 113 is in a second rotational position shown in Fig. 5. Thus, depending on the rotational position of the longitudinal expansion element 113 the cover 108 expands or contracts.

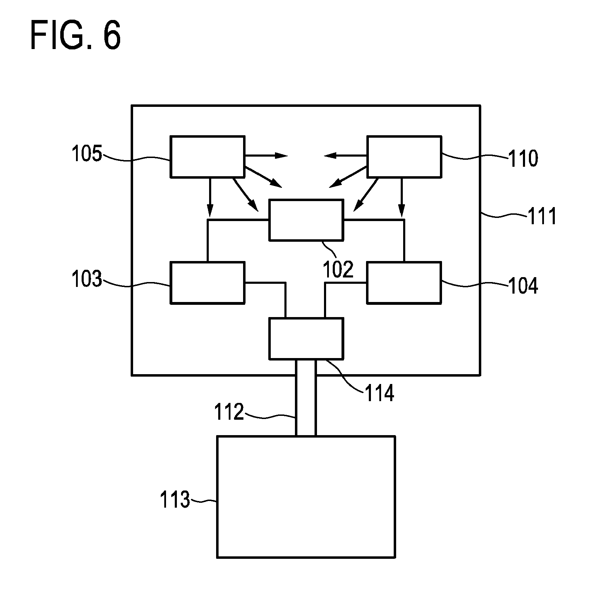

Fig. 6 shows schematically and exemplarily the units within the compartment 111.

The shaft 112 is connected with a rotating motor 114 for rotating the rotatable shift 112 and, thus, the longitudinal expansion element 113. The respiration controlling apparatus 101 comprises the heart rate sensing unit 102 with the sensing region 109 for generating a heart rate signal indicative of the heart rate of the person, the breathing sequence determining unit 103 for determining a desired breathing sequence based on the generated heart rate signal and the coherence determining unit 104 for determining a degree of coherence. The breathing sequence determining unit 103 and the coherence determining unit 104 are connected with the rotating motor 114 for modifying the rotational position of the longitudinal expansion element 113 and, thus, the shape of the respiration controlling apparatus 101 depending on the desired breathing sequence and/or the determined degree of coherence. The respiration controlling apparatus 101 further comprises an energy source like a rechargeable battery for providing the different units of the respiration controlling apparatus 101 with electrical energy and a control unit 110 for controlling the several units within the compartment 111.

The rotating motor 114, the shaft 112, the longitudinal expansion element 113 and the expansible cover 108 form a haptic output unit.

The expansible cover 108 is preferentially an expansible plastic cover. The expansible cover is preferentially soft, easy to clean, and not fragile to prevent damage, if the respiration controlling apparatus falls out of a bed. The expansible cover can, for example, be made of a rubber material.

In an embodiment, the control unit comprises a timer and controls the respiration controlling apparatus such that the respiration controlling apparatus is switched into an idle mode, if the respiration controlling apparatus has not been used for a predefined time. This idle mode saves power and can ensure that a user, who gets into a sleep stage, is not disturbed by the respiration controlling apparatus. Preferentially, in the idle mode the energy source is completely switched off or the energy source provides at least less energy in comparison to a full operation mode.

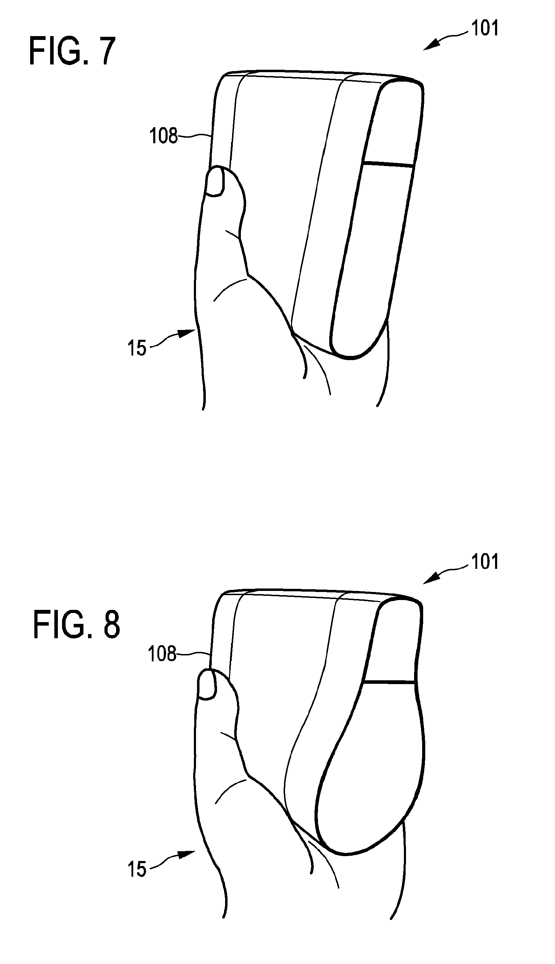

Fig. 7 shows schematically and exemplarily the respiration controlling apparatus 101 held by a hand 15 of a person. In Fig. 7, the respiration controlling apparatus 1 is contracted, i.e. it corresponds to the situation shown in Fig. 5. In Fig. 8, the respiration controlling apparatus is expanded, i.e. the situation shown in Fig. 8 corresponds to the situation shown in Fig. 4.

The respiration controlling apparatus 101 is preferentially adapted to expand at the inhale times and to contract at the exhale times. Moreover, the respiration controlling apparatus 101 can be adapted to contract, i.e. to decrease its volume, if the degree of coherence increases.

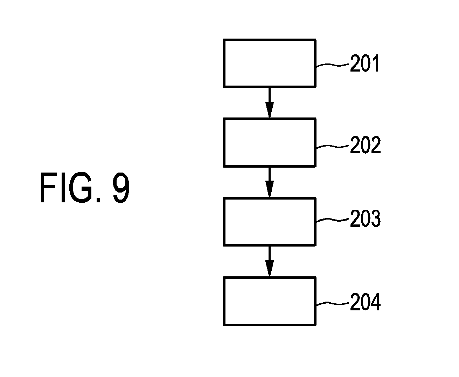

In the following a respiration controlling method for controlling the respiration of a person will exemplarily and schematically be described with reference to a flowchart shown in Fig. 9.

In step 201, the respiration controlling apparatus is arranged on a hand of a person, wherein a finger tip of the person is located at the sensing area of a heart rate sensing unit.

In step 202, a heart rate signal is generated by the heart rate sensing unit, and in step 203 a desired breathing sequence is determined based on the generated heart rate signal.

In step 204, the haptic output unit outputs haptic output signals depending on the determined desired breathing sequence.

In an embodiment, in step 203 a degree of coherence can be determined, wherein in this case in step 204 the haptic output unit can output haptic output signals depending on the determined degree of coherence.

Biofeedforward and biofeedback with heart-rate variability can be used to improve sleep problems. The heart rate variability is attributed to the balance between the parasympathetic and the sympathetic nervous system, respectively decreasing and increasing the heart rate. Biofeedforward and biofeedback can be used in clinical applications, but also in a private environment, for example, in order to improve sleep quality in general and in particular decreasing the sleep-onset time. Thus, the respiration controlling apparatus can be used, for example, in clinical applications or in a private environment for, for example, improving the sleep quality, relaxation or in other fields like sports in general and lowering the blood pressure in particular. The respiration controlling apparatus is preferentially adapted to provide voluntary cardio respiratory synchronization (VCRS) by using haptic, i.e. tactile and/or proprioception, outputs and by giving biofeedforward and preferentially biofeedback about the level of coherence to the person. The respiration controlling apparatus does not disturb, for example, a bed partner or the person using the respiration controlling apparatus, because preferentially it does not output noise or distracting lights.

For biofeedback and biofeedforward systems the user needs to get an indication of some measure or action. In the case of biofeedback the user needs to get feedback on, for example, the heart rate variability. In the case of feedforward systems, the user needs to receive, for example, breathing pace commands for commanding a breathe in and/or a breathe out. The respiration controlling apparatus contains several blocks being, in particular, sensing the heart rate, determining a desired breathing sequence, and outputting a haptic signal either as a feedback or as a feedforward.

Although in the above described embodiments the heart rate sensing unit is a PPG sensor, in other embodiments, another heart rate sensing unit can be used. For example, electrodes can be used for measuring an electrocardiogram signal. Moreover, a ballistocardiogram can be measured, for example, with a static charge sensitive bed, a piezo foil or an EMFi-film sensor build into a chair as disclosed in, for example, "An EMFi-film sensor based ballistocardiographic chair performance and cycle extraction method", S. Junnila, A. Akhbardeh, A. Varri, T. Koivistoinen; IEEE Workshop on Signal Processing Systems Design and Implementation, published 2-4 November 2005, pages 373-377. Moreover, the heart rate sensing unit can be a unit for measuring the oxygen saturation (SP02). Furthermore, instead of measuring the PPG signal, i.e. the photo-plethysmogram signal, a non-photo-plethysmogram signal can be measured. The heart rate sensing unit can also use non-galvanic capacitive electrodes as disclosed in "High resolution ambulatory electrocardiographic monitoring using wrist-mounted electric potential sensors", C.J. Harland, T.D. Clark and R.J. Prance, Meas. Sci. Technol, volume 14, number 7, 2003, pages 923-928, a wristwatch like device as disclosed in WO 2007/072239 A2, a seismosomnography unit as disclosed in "Contact-free measurement of heart rate, respiration rate, and body movements during sleep", M. Brink, C.H. Mueller, C. Schierz, Behavior Research Methods 2006, 38(3), pages 511-521, an ultra wide band radar, an optical vibrocardiography apparatus as disclosed in "Optical Vibrocardiography: A novel tool for the optical Monitoring of Cardiac Activity", U. Morbiducci, L. Scalise, M. Melis, M. Grigioni, Annals of Biomedical Engineering, volume 35(1), pages 45-58, January 2007, an acoustical unit for acoustically sensing the heart rate via a microphone also known as phonocardiograph, et cetera.

Although in above described embodiments a finger is placed at a sensing area for sensing the heart rate, in other embodiments the heart rate can be sensed at another part of the person, for example, at the ear.

Although in the above described embodiments, the respiration controlling apparatus is a handheld apparatus, it can also be another kind of apparatus like a standalone apparatus or like an apparatus which is integrated into textile, in particular, into underwear.

The respiration controlling apparatus is preferentially adapted such that the measurement of the heart rate is unobtrusive.

The respiration controlling apparatus is preferentially adapted to determine the inter-beat intervals from the generated heart rate signal as the time between the R peaks, i.e. preferentially the temporal position of a heart beat is determined by an R peak.

The respiration controlling apparatus can be adapted to provide to a person with VCRS cues by outputting the haptic output signals. Moreover, the respiration controlling apparatus can be adapted to use additionally scent for giving VCRS cues.

Although in the above described embodiments certain haptic output units have been described for outputting haptic output signals, in other embodiments other haptic output units can be used for outputting the haptic output signals. For example, the haptic output unit can be adapted to provide vibrations to a person, wherein vibrating motors can be integrated in, for example, a bedding or a pillow. In an embodiment, the haptic output unit is adapted to provide vibrations on the back of the person, wherein the vibrations move towards the head for indicating inhaling times and towards the bottom for indicating exhaling times. Moreover, the haptic output unit can be a handheld device which incorporates a speaker that is behind a structure that can open and close, in particular, similar to the opening and closing of a flower. The speaker is preferably revealed and closed based on VCRS, thus combining both audio and haptic, in particular, tactile, signals. Moreover, the haptic output unit can be adapted to provide haptic output signals by heating or cooling a region that can be in touch with the person. The heating can also be used to just warm body parts of the person like the hands. The respiration controlling apparatus can also be adapted to use the temperature change for giving feedback. For example, if the degree of coherence increases, also the temperature can be increased.

In addition to the haptic output unit, the respiration controlling apparatus can comprise an audio unit for providing audio signals. For example, an audio setup can be provided in a bedroom, which has speakers at both ends, i.e. head end and feet end, of a bed. The location of the auditory event provides feedforward and can be synchronized with the haptic feedforward, for example, when the auditory event is at the feet end, the user should breathe out, and when it is at the head end, the user should inhale. The auditory event location can fluently move from one end to the other. Moreover, the audio unit can be adapted to provide coherence feedback, for example, when the degree of coherence is low, the auditory event can be at the feet end, and, when it is at the head end, the degree of coherence can be larger. The auditory event location can be fluently moved from one end to the other end as the user becomes more or less coherent.

Furthermore, the respiration controlling apparatus can consist of two separate entities so that each of these entities can be held in a hand of a person. The two entities can then provide the same haptic output signals at the same times, i.e. they can be synchronized. Or they can provide haptic output signals at different times, in particular, they can provide different haptic output signals. For example, the entity in one hand can be adapted to provide paced breathing cues, i.e. haptic output signals for indicating inhale times and exhale times to a person, while the entity in the other hand can be adapted to provide a feedback about the degree of coherence.

In a further embodiment, the respiration controlling apparatus can be adapted to change its overall shape for providing haptic output signals. For example, the respiration controlling apparatus can be adapted such that the overall shape starts to have sharp edges, if the degree of coherence decreases, whereas these edges fade out for forming a more rounded shape, if the degree of coherence increases. The respiration controlling apparatus can be adapted to detect if the person has fallen asleep, in particular, from the sensed heart rate signal, or to detect that the user has left the device, i.e. that the person does not touch the respiration controlling apparatus anymore. The respiration controlling apparatus can be adapted to undertake then appropriate actions, in particular, to turn itself off.

The respiration controlling apparatus is preferably used to relax the person in general. In particular, the respiration controlling apparatus can be adapted to decrease the sleep-on set time, to perform yoga exercises, to lower the blood pressure, to relax during PC-use or television watching, vehicle driving, medical procedures like surgical procedures, and sports.

Although in the above described embodiments the coherence determining unit is adapted to determine the degree of coherence depending on an amplitude of an HRV signal, in other embodiments the coherence determining unit can be adapted to determine a degree of coherence by other known coherence determining methods.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the generation of the heart rate signal, the determination of the desired breathing sequence, et cetera, performed by one or several units or devices can be performed by any other number of units or devices. For example, the determination of the desired breathing sequence and the degree of coherence can be performed by a single unit or by any other number of different units. The different procedures of the respiration controlling apparatus like the determination of the desired breathing sequence and/or the determination of a degree of coherence, and/or the control of the respiration controlling apparatus in accordance with the respiration controlling method can be implemented as program code means of a computer program and/or a dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a respiration controlling apparatus for controlling the respiration of a person. The respiration controlling apparatus comprises a heart rate sensing unit for generating a heart rate signal indicative of the heart rate of the person, a breathing sequence determining unit for determining a desired breathing sequence based on the generated heart rate signal, and a haptic output unit for outputting haptic output signals depending on the determined desired breathing sequence. The respiration controlling apparatus is preferentially used for providing a biofeedforward to a person. Since haptic signals are output, signals like biofeedforward signals can be provided to a person without disturbing another person. This increases the application area of the respiration controlling apparatus.

**Claims**

1.  A respiration controlling apparatus for controlling the respiration of a person, the respiration controlling apparatus (1;101) comprising:

    - a heart rate sensing unit (2; 102) for generating a heart rate signal indicative of the heart rate of the person,
    - a breathing sequence determining unit (3; 103) for determining a desired breathing sequence (16) based on the generated heart rate signal,
    - a haptic output unit (6, 7, 8; 108, 113, 112, 114) for outputting haptic output signals depending on the determined desired breathing sequence (16).

2.  The respiration controlling apparatus as defined in claim 1,wherein the haptic output unit (6, 7, 8; 108, 113, 112, 114) is adapted to haptically instruct the person to breathe in and to breathe out in accordance with the desired breathing sequence (16).

3.  The respiration controlling apparatus as defined in claim 2, wherein the breathing sequence determining unit (3; 103) is adapted to determine inhale times (p) and exhale times (q) as the breathing sequence (16) and wherein the haptic output unit (6, 7, 8; 108, 113, 112, 114) is adapted to haptically output the inhale times (p) and the exhales times (q) to the person.

4.  The respiration controlling apparatus as defined in claim 3, wherein the haptic output unit (6, 7, 8; 108, 113, 112, 114) is adapted to output first haptic signals at the inhale times (p) and second haptic signals at the exhale times (q), wherein the first and second haptic signals are different.

5.  The respiration controlling apparatus as defined in claim 1, wherein the respiration controlling apparatus (1; 101) further comprises a coherence determining unit (4; 104) for determining a degree of coherence depending on the generated heart rate signal and wherein the haptic output unit (6, 7, 8; 108, 113, 112, 114) is adapted to provide a haptic output signal depending on the determined degree of coherence.

6.  The respiration controlling apparatus as defined in claim 1, wherein the respiration controlling apparatus (1; 101) is a handheld apparatus.

7.  The respiration controlling apparatus as defined in claim 1, wherein the haptic output unit (108, 113, 112, 114) is adapted to expand and to contract for outputting haptic output signals.

8.  The respiration controlling apparatus as defined in claim 1, wherein the haptic output unit (6, 7, 8) is adapted to move for outputting a haptic output signal.

9.  The respiration controlling apparatus as defined in claim 8, wherein the haptic output unit (6, 7, 8) comprises a movable weight (6) and a weight moving unit (7) for moving the weight (6) such that a balance point of the haptic output unit (6, 7, 8) is modified for moving the haptic output unit.

10. The respiration controlling apparatus as defined in claim 1, wherein the haptic output unit (6, 7, 8; 108, 112, 113, 114) comprises an outer casing (8; 108) including at least the breathing sequence determining unit (3; 103) for determining a desired breathing sequence based on the generated heart rate signal.

11. The respiration controlling apparatus as defined in claim 10, wherein the outer casing (8) is spherical.

12. A respiration controlling method for controlling the respiration of a person, the respiration controlling method comprising following steps:

- generating a heart rate signal indicative of the heart rate of the person,
- determining a desired breathing sequence (16) based on the generated heart rate signal,
- outputting haptic output signals depending on the determined desired breathing sequence.

13. A respiration controlling computer program for controlling the respiration of a person, the respiration controlling computer program comprising program code means for causing a respiration controlling apparatus (1; 101) as defined in claim 1 to carry out the steps of the respiration controlling method as defined in claim 12, when the computer program is run on a computer controlling the respiration controlling apparatus (1; 101).

## FIG. 1

## FIG. 2

FIG. 3

**FIG. 4**

109

101

111

112

108

113

**FIG. 5**

109

101

111

112

108

113

FIG. 6

FIG. 7

101

108

15

FIG. 8

101

108

15

FIG. 9

201

202

203

204

Europäisches
Patentamt

European
Patent Office

Office européen
des brevets

## EUROPEAN SEARCH REPORT

Application Number

EP 09 17 2859

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/110956 A1 (KONINKL PHILIPS ELECTRONICS NV [NL]; WESTERINK JOANNE H D M [NL]; OVER) 18 September 2008 (2008-09-18) * page 2, line 11 - line 18 * * page 6, line 10 - line 17 * * page 11, line 1 - line 34; figures * | 1-7,9-13 | INV. A63B23/18 A41D13/00 A61B5/00 G06F3/01 |
| X | US 2005/240114 A1 (ELLIOTT STEPHEN B [US]) 27 October 2005 (2005-10-27) * paragraph [0020]; figures * * paragraph [0008] * | 1-13 | |
| X | US 5 395 301 A (RUSSEK LINDA G [US]) 7 March 1995 (1995-03-07) * the whole document * | 1-4,6,8, 12-13 | |

| | | | TECHNICAL FIELDS SEARCHED (IPC) |
|---|---|---|---|
| | | | A63B A41D A61B G06F |

The present search report has been drawn up for all claims

| Place of search | Date of completion of the search | Examiner |
|---|---|---|
| Munich | 4 March 2010 | Squeri, Michele |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    ...........................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 09 17 2859

This annex lists the patent family members relating to the patent documents cited in the above-mentioned European search report.
The members are as contained in the European Patent Office EDP file on
The European Patent Office is in no way liable for these particulars which are merely given for the purpose of information.

04-03-2010

| Patent document cited in search report | | Publication date | Patent family member(s) | | Publication date |
|---|---|---|---|---|---|
| WO 2008110956 | A1 | 18-09-2008 | EP | 1988480 A1 | 05-11-2008 |
| US 2005240114 | A1 | 27-10-2005 | NONE | | |
| US 5395301 | A | 07-03-1995 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007072239 A2

**Non-patent literature cited in the description**

- **S. Junnila ; A. Akhbardeh ; A. Varri ; T. Koivistoinen.** An EMFi-film sensor based ballistocardiographic chair performance and cycle extraction method. *IEEE Workshop on Signal Processing Systems Design and Implementation,* 02 November 2005, 373-377
- **C.J. Harland ; T.D. Clark ; R.J. Prance.** High resolution ambulatory electrocardiographic monitoring using wrist-mounted electric potential sensors. *Meas. Sci. Technol,* 2003, vol. 14 (7), 923-928
- **M. Brink ; C.H. Mueller ; C. Schierz.** Contact-free measurement of heart rate, respiration rate, and body movements during sleep. *Behavior Research Methods,* 2006, vol. 38 (3), 511-521
- Optical Vibrocardiography: A novel tool for the optical Monitoring of Cardiac Activity. **U. Morbiducci ; L. Scalise ; M. Melis ; M. Grigioni.** Annals of Biomedical Engineering. January 2007, vol. 35, 45-58